(19) Europäisches Patentamt
European Patent Office
Office européen des brevets

(11) Numéro de publication: **0 281 459 B1**

(12) **FASCICULE DE BREVET EUROPEEN**

(45) Date de publication de fascicule du brevet: **26.04.95**

(51) Int. Cl.[6]: **C07D 495/04**, C07B 57/00, A61K 31/445, C07J 41/00, A61K 31/575, //(C07D495/04, 333:00,221:00)

(21) Numéro de dépôt: **88400346.8**

(22) Date de dépôt: **16.02.88**

Le dossier contient des informations techniques présentées postérieurement au dépôt de la demande et ne figurant pas dans le présent fascicule.

(54) **Enantiomère dextrogyre de l'alpha-(tétrahydro-4,5,6,7 thiéno (3,2-c)pyridyl-5) (chloro-2 phényl)-acétate de méthyle, son procédé de préparation et les compositions pharmaceutiques le renfermant.**

(30) Priorité: **17.02.87 FR 8702025**
**27.11.87 FR 8716516**

(43) Date de publication de la demande:
**07.09.88 Bulletin 88/36**

(45) Mention de la délivrance du brevet:
**26.04.95 Bulletin 95/17**

(84) Etats contractants désignés:
**AT BE CH DE ES FR GB GR IT LI LU NL SE**

(56) Documents cités:
**EP-A- 0 099 802**

(73) Titulaire: **SANOFI**
**32-34, rue Marbeuf**
**F-75008 Paris (FR)**

(72) Inventeur: **Badorc, Alain**
**1 Rue La Canal**
**F-31120 Roquettes (FR)**
Inventeur: **Frehel, Daniel**
**Résidence l'Autan**
**100 Allée de Barcelone**
**F-31000 Toulouse (FR)**

(74) Mandataire: **Polus, Camille et al**
**c/o Cabinet Lavoix**
**2, Place d'Estienne d'Orves**
**F-75441 Paris Cedex 09 (FR)**

**EP 0 281 459 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à un sel de l'énantiomère dextrogyre de l'alpha-(tétrahydro-4,5-,6,7 thiéno (3,2-c) pyridyl -5) (chloro-2 phényl)-acétate de méthyle, à son procédé de préparation et aux compositions pharmaceutiques le renfermant.

Cet énantiomère répond à la formule (I) suivante :

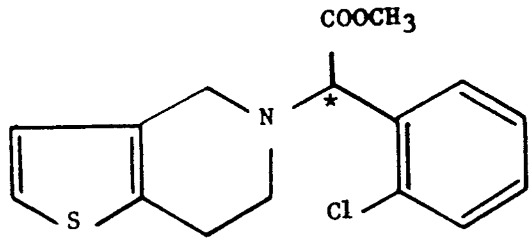

(I)

dans laquelle le carbone C* est un carbone asymétrique. En fait, cette formule représente aussi bien la molécule dextrogyre que son énantiomère lévogyre. Le mélange racémique répondant à cette formule a été décrit dans la demande de brevet français publié sous le n° 2 530 247. On désignera ci-après par $I_d$ l'énantiomère dextrogyre et comme $I_\ell$ l'énantiomère lévogyre.

On sait que le pouvoir rotatoire d'un composé dépend du solvant dans lequel il est mesuré et de sa concentration dans ce solvant. L'isomère dextrogyre selon l'invention a un pouvoir rotatoire positif en solution dans le méthanol.

De façon inattendue, seul l'énantiomère dextrogyre $I_d$ présente une activité anti-agrégante plaquettaire, l'énantiomère lévogyre $I_\ell$ étant inactif. Par ailleurs, l'énantiomère lévogyre $I_\ell$ inactif est le moins bien toléré des deux énantiomères.

Le composé ($I_d$) est une huile, tandis que son chlorhydrate se présente sous forme d'une poudre blanche. Les produits huileux sont généralement difficiles à purifier, et on préfère utiliser pour la préparation de compositions pharmaceutiques des produits cristallins qui peuvent généralement être purifiés par recristallisation.

On a toutefois constaté dans le cas présent, que certains sels du composé ($I_d$) précipitent généralement sous forme amorphe et/ou qu'ils sont hygroscopiques, ce qui rend délicate leur manipulation au stade industriel. On a donc préparé les sels d'acides carboxyliques et sulfoniques classiques en pharmacie, tels que les acides acétique, benzoique, fumarique, maléique, citrique, tartrique, gentisique, méthanesulfonique, éthanesulfonique, benzènesulfonique, et laurylsulfonique, ainsi que le sel de l'acide dobésilique (F = 70 °C), et celui de l'acide para-toluènesulfonique (F = 51 °C), dont la purification s'est avérée délicate.

On a trouvé parmi les sels d'acides organiques et minéraux de l'isomère dextrogyre du composé de formule $I_d$, des sels qui cristallisent facilement, ne sont pas hygroscopiques et sont suffisamment hydrosolubles pour que leur utilisation comme principes actifs de médicament soit particulièrement avantageuse.

La présente invention concerne donc plus particulièrementl'hydrogénosulfate de l'énantiomère dextrogyre de l'alpha-(tétrahydro-4,5,6,7 thiéno(3,2-c) pyridyl-5) (chloro-2 phényl)-acétate de méthyle.

Ce sel est préparé de façon classique par action de l'acide correspondant sur la base en solution dans un solvant dans lequel il précipite spontanément ou après addition d'un non-solvant du sel.

L'isomère dextrogyre de l'alpha-(tétrahydro-4,5,6,7 thiéno (3,2-c) pyridyl-5)(chloro-2 phényl)-acétate de méthyle peut être préparé par salification du composé racémique par un acide optiquement actif dans un solvant, recristallisations successives du sel jusqu'à obtention d'un produit de pouvoir rotatoire constant, puis libération de l'isomère dextrogyre de son sel par une base ; le cas échéant l'isomère dextrogyre est salifié avec un acide pharmaceutiquement acceptable.

L'acide optiquement actif peut être avantageusement l'acide camphosulfonique-10 lévogyre.

On peut utiliser un seul solvant pour la salification et la recristallisation : dans ce cas, l'acétone convient parfaitement.

L'acide camphosulfonique-10 chiral lévogyre deformule ($II_l$) est mis à réagir dans un solvant inerte avec le mélange racémique de formule (I) selon le schéma réactionnel suivant :

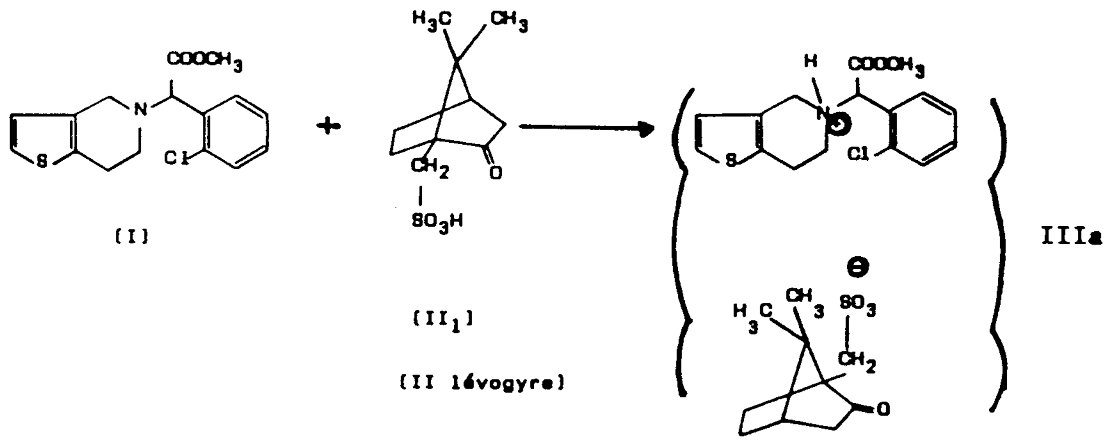

(I) (II$_l$) (II lévogyre) IIIa

La salification peut être réalisée dans des solvants tels que les alcools, les cétones, le diméthylformamide. Le sel précipite spontanément ou est isolé par relargage ou évaporation du solvant. Il se forme un mélange des deux diastéréoisomères de formule (IIIa). Par recristallisations successives dans un solvant comme l'acétone, le précipité est enrichi en sel de l'isomère dextrogyre du composé (I). Après chaque recristallisation, le pouvoir rotatoire $[\alpha]_D^{20}$ du précipité est mesuré a 20 °C dans le méthanol, à une concentration comprise entre 1,5 et 2 g/100 ml. Dès que $[\alpha]_D^{20}$ n'évolue plus, on libère la base de formule ($I_d$) à partir du sel (IIIa), par action d'une base telle que l'hydrogénocarbonate de sodium ou de potassium en milieu aqueux à des températures comprises entre 5 °C et 20 °C.

L'évaporation du filtrat de la 1ère recristallisation IV après filtration des cristaux du sel précipité (IIIa) donne un mélange enrichi en sel de l'énantiomère ($I_\ell$). L'alcalinisation de ce mélange de sels diastéréoisomériques, avec une base faible telle que l'hydrogénocarbonate de sodium ou de potassium, en solution aqueuse, à des températures comprises entre 5 °C et 20 °C, conduit à un mélange des deux énantiomères ($I_d$) + ($I_l$), enrichi en énantiomère lévogyre ($I_\ell$).

On fait réagir ce mélange ($I_d$) + ($I_\ell$) enrichi en énantiomère ($I_\ell$) avec l'acide camphosulfonique-10 dextrogyre, que nous désignerons comme ($II_d$), dans un solvant, selon le schéma réactionnel suivant :

**($I_d$) + ($I_l$) +**
**avec ($I_l$) majoritaire**

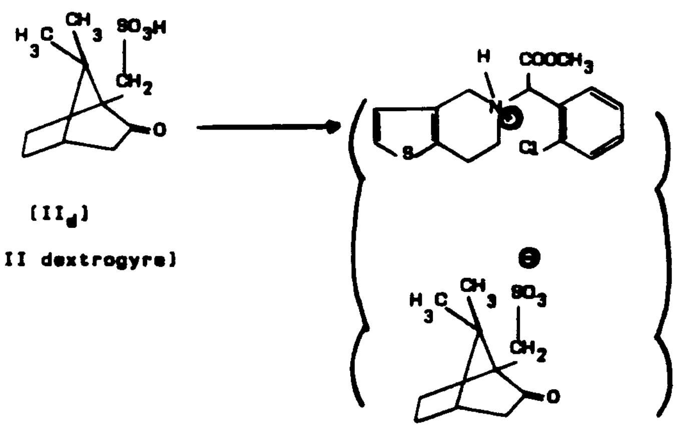

($II_d$) (II dextrogyre) IIIb

Le mélange de sels diastéréoisomériques (IIIb) cristallin, obtenu, est recristallisé dans l'acétone jusqu'à ce que le pouvoir rotatoire $[\alpha]_D^{20}$ du précipité soit constant. On détermine comme précédemment le pouvoir rotatoire $[\alpha]_D^{20}$ du sel diastéréoisomérique ($III_b$) après chaque recristallisation.

La libération du stéréoisomère ($I_\ell$) de son sel est effectuée de façon classique, comme celle du composé (Id).

L'acide camphosulfonique-10 lévogyre ($II_\ell$) peut être obtenu à partir du camphosulfonate-10 d'ammonium commercial de formule (V) selon le schéma réactionnel :

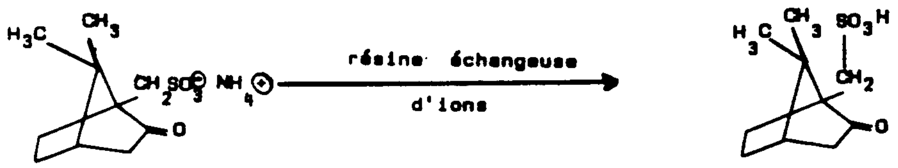


(V) (IIℓ)

On chromatographie une solution aqueuse du sel d'ammonium (V) sur une résine Amberlite IRN-77. Après lyophilisation de l'éluat, on récupère l'acide camphosulfonique-10, de formule ($II_ℓ$).

L'ensemble du procédé est schématisé ci-dessous :

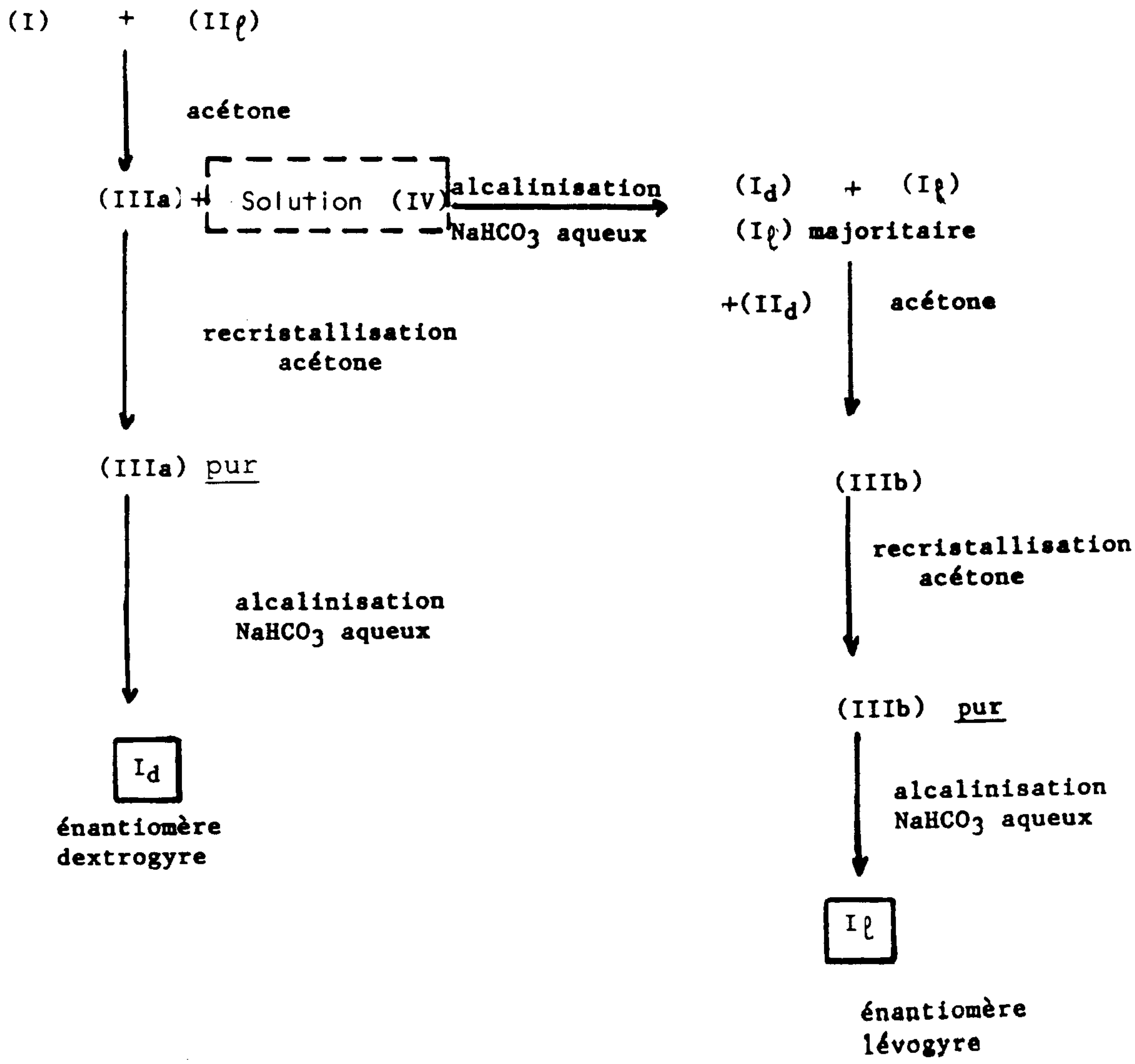


Chacun des énantiomères ($I_d$) et ($I_ℓ$) pur peut être salifié suivant les méthodes classiques : par exemple les chlorhydrates sont préparés par addition d'une solution de gaz chlorhydrique dans l'éther diéthylique à une solution de ($I_d$) ou ($I_ℓ$) dans l'éther diéthylique.

Détermination de la pureté énantiomérique des énantiomères dextrogyre ($I_d$) et lévogyre ($I_\ell$)

Deux méthodes ont été utilisées :

- spectroscopie RMN du proton avec adjonction de terre rare chirale
- chromatographie liquide haute pression utilisant une phase stationnaire chirale.

**a) spectroscopie RMN du proton avec adjonction de terre rare chirale**

La pureté énantiomérique (pureté optique) a été déterminée par spectroscopie RMN$^1$H 60 MHz, en présence de complexe chiral de terre rare, selon la méthode décrite par G.M. WHITESIDES et al. (J. Am. Chem. Soc. 1974, 96, 1038).

Sur le produit racémique (I), l'hydrogène fixé sur le centre d'asymétrie, en $\alpha$ de la fonction ester, apparaît comme un singulet (déplacement chimique $\delta$ = 4,87 ppm, dans $CDCl_3$ comme solvant. L'adjonction du complexe de terre rare $Eu(tfc)_3$ [tris (trifluorométhyl hydroxyméthylène-3)-d-camphorate d'europium (III)] dans la sonde, contenant la solution du racémique (I) dans $CDCl_3$, conduit au dédoublement du singulet initial en deux singulets bien séparés, correspondant au proton de chacun des deux énantiomères ($I_d$) et ($I_\ell$). Pour un rapport molaire complexe terre rare/composé (I) = 0.4, la séparation entre les deux singulets est de 6Hz.

Avec chacun des deux énantiomères préparés ($I_d$) et ($I_\ell$), la même procédure que pour le racémique (I) a été utilisée. Le déplacement chimique le plus faible correspond au proton de l'énantiomère dextrogyre ($I_d$) et le déplacement chimique le plus élevé à l'énantiomère lévogyre ($I_\ell$).

La précision de la méthode a été déterminée en comparant les spectres de RMN$^1$H (60 MHz), obtenus avec ou sans adjonction de complexe de terre rare, pour chacun des deux énantiomères ($I_d$) et ($I_\ell$), purs ou en mélange avec des quantités croissantes de l'autre énantiomère. On a constaté qu'il était possible de détecter facilement, plus de 5 % en poids de l'un des énantiomères en mélange avec l'autre.

**b) chromatographie liquide haute pression utilisant une phase stationnaire chirale**

L'étude a été conduite avec un chromatographe liquide HP-1084 en utilisant un détecteur UV à 215 nm. La phase stationnaire chirale était de la silice DEAE (10 microns), greffée avec une alpha-1 glycoprotéine acide (0,4 x 100 mm) (ENANTIOPAC R-LKB). La phase mobile étant constituée d'un mélange aqueux tampon phosphate ($NaH_2PO_4/Na_2HPO_4$)-8mM, contenant 0,1 M de NaCl, ajusté à pH = 7,4 et contenant 15% d'isopropanol (V/V). Le débit a été fixé à 0,3 ml/minute et la température de la colonne a été maintenue vers 18-20 ° C.

Dans ces conditions, l'énantiomère dextrogyre ($I_d$) a un temps de rétention de 45 minutes et l'énantiomère lévogyre ($I_\ell$) a un temps de rétention de 35 minutes.

La précision de la détermination de la pureté optique des deux énantiomères a été estimée en chromatographiant chacun des deux énantiomères ($I_d$) et ($I_\ell$) préparés, soit seul, soit en mélange avec des quantités croissantes de l'autre énantiomère. On a constaté qu'il était possible de détecter facilement :

- 2 % (poids/poids) d'énantiomère ($I_d$) dans l'énantiomère ($I_\ell$)
- 4 % (poids/poids) d'énantiomère ($I_\ell$) dans l'énantiomère ($I_d$).

Dans ces conditions, on peut conclure que la pureté optique des deux énantiomères ($I_d$) et ($I_\ell$) obtenus selon les exemples est au moins égale à 96 % pour l'énantiomère dextrogyre ($I_d$) et au moins égale à 98 % pour l'énantiomère lévogyre ($I_\ell$).

Les exemples non limitatifs suivants sont donnés à titre d'illustration de la présente invention.

EXEMPLE 1 - Sels de l'alpha-(tétrahydro-4,5,6,7 thiéno(3,2-c) pyridyl-5) (chloro-2 phényl)-acétate de méthyle dextrogyre.

a) Acide camphosulfonique-10 lévogyre

On prépare une colonne de résine Amberlite IRN-77, qu'on traite par passage d'acide chlorhydrique 1N, et on lave cette colonne de résine acidifiée par de l'eau, abondamment. On dissout le camphosulfonate-10 d'ammonium lévogyre dans le minimum d'eau et le dépose sur la colonne de résine, préalablement préparée. On élue avec de l'eau. Les fractions d'élution contenant l'acide sont lyophilisées.

Cristaux blancs, F = 198 ° C; $[\alpha]_D^{20}$ = -20,53 (C : 2,075 g/100 ml, eau).

b) sel de l'acide 1-camphosulfonique-10 de l'alpha-(tétrahydro-4,5,6,7 thiéno(3,2-c) pyridyl-5) (chloro-2 phényl)- acétate de méthyle(SR 25990 B).

On dissout 32 g (0,0994 mole) de l'alpha-(tétrahydro-4,5,6,7 thiéno(3,2-c) pyridyl-5) (chloro-2 phényl)-acétate de méthyle racémique dans 150 ml d'acétone. On ajoute 9,95 g (0,0397 mole) d'acide camphosulfonique-10 monohydraté lévogyre. Le milieu homogène est abandonné à température ambiante. Après 48 heures, il y a apparition de quelques cristaux. On concentre le milieu réactionnel à 50 ml et abandonne 24 heures à température ambiante. Les cristaux obtenus sont filtrés, lavés à l'acétone et séchés (Rdt. : 55% par rapport au racémique de départ).

Cristaux blancs, F = 165 °C, $[\alpha]_D^{20}$ = + 24,67 (C = 1,58 g/100 ml; méthanol).

Les cristaux obtenus précédemment sont redissous dans le minimum d'acétone bouillante (50 ml). Les cristaux obtenus après refroidissement sont filtrés, lavés à l'acétone et séchés (rendement : 88%).

Cristaux blancs, F = 165 °C, $[\alpha]_D^{20}$ = + 24,75 (C = 1,68 g/100 ml; méthanol).

c) Alpha-(tétrahydro-4,5,6,7 thiéno(3,2-c) pyridyl-5) (chloro-2 phényl)- acétate de méthyle dextrogyre.

On dissout 12 g (0,022 mole) du produit pur obtenu en b), dans le minimum d'eau. En refroidissant à 5 °C, on alcalinise la solution aqueuse obtenue avec une solution aqueuse saturée d'hdyrogénocarbonate de sodium. On extrait la phase aqueuse alcaline avec du dichlorométhane. Les extraits organiques sont séchés sur du sulfate de sodium anhydre. L'évaporation du solvant laisse une huile incolore (rendement quantitatif). Huile, $[\alpha]_D^{20}$ = + 51,52 (C = 1,61 g/100 ml; méthanol).

d) Hydrogénosulfate de l'alpha-(tétrahydro-4,5,6,7 thiéno(3,2-c) pyridyl-5) (chloro-2 phényl)-acétate de méthyle dextrogyre (SR 25990 C).

On ajoute 800 ml d'une solution aqueuse saturée de bicarbonate de sodium à une suspension de 200 g de SR 25990 B dans 800 ml de dichlorométhane. Après agitation, la phase organique est décantée, séchée sur sulfate de sodium et le solvant éliminé sous pression réduite. Le résidu est dissous dans 500 ml d'acétone refroidi dans la glace et on ajoute goutte à goutte 20,7 ml d'acide sulfurique concentré (93,64 % -d = 1,83). Le précipité apparu est isolé par filtration et lavé avec 1000 ml d'acétone puis séché en étuve à vide à 50 °C.

On obtient ainsi 139 grammes de cristaux blancs, analytiquement purs de point de fusion 184 °C. Formule brute : $C_{16}H_{16}ClNO_2S,H_2SO_4$

$$[\alpha]_D^{20\,°C} = + 55,10$$

(C = 1,891 g/100 ml, méthanol)

<u>EXEMPLE 2</u> - Sels l'alpha-(tétrahydro-4,5,6,7 thiéno(3,2-c) pyridyl-5) (chloro-2 phényl)-acétate de méthyle lévogyre.

a) Sel de l'acide d-camphosulfonique-10 (SR 25989 B)

On évapore le solvant de la solution acétonique obtenue à l'exemple 1-b, après séparation du SR 25990 B.

On reprend le résidu par de l'eau et de l'éther diétylique. La phase éthérée est décantée. La phase aqueuse est refroidie à 5 °C et alcalinisée avec une solution aqueuse saturée de bicarbonate de sodium. On extrait la phase aqueuse alcaline avec de l'éther diéthylique. Les extraits éthérés sont joints et séchés sur du sulfate de sodium anhydre.

L'évaporation laisse une huile que l'on purifie par filtration sur un lit de silice (éluant : éther diéthylique).

On récupère une huile incolore, composée d'un mélange d'environ 65% de l'énantiomère lévogyre et 35% de l'énantiomère dextrogyre, proportions déterminées par spectroscopie RMN$^1$H (60 MHz) avec l'adjonction d'un complexe de terre rare chirale.

On dissout 16,66 g (0,0517 mole) du mélange ainsi obtenu dans 70 ml d'acétone. On y ajoute 7,77 g (0,0310 mole) d'acide camphosulfonique-10 monhydraté dextrogyre. Le milieu homogène est abandonné une nuit à température ambiante. Les cristaux obtenus sont filtrés, lavés à l'acétone et séchés (Rdt. : 44%

par rapport au mélange).

Les cristaux obtenus sont dissous dans le minimum d'acétone au reflux (60 ml). Le précipité obtenu après refroidissement à température ambiante est filtré, lavé a l'acétone et séché. Cristaux blancs, F = 167 ° C, $[\alpha]_D^{20}$ = - 24,85 (C = 1,79 g/100 ml, méthanol).

b) Alpha-(tétrahydro-4,5,6,7 thiéno(3,2-c) pyridyl-5) (chloro-2 phényl)-acétate de méthyle lévogyre.

On dissout 11,3 g (0,0204 mole) du camphosulfonate-10, obtenu en a), dans le minimum d'eau. En refroissant à 5 ° C, on alcalinise la solution aqueuse obtenue avec une solution aqueuse saturée d'hydrogénocarbonate de sodium. On extrait la phase aqueusealcaline avec du dichlorométhane. La solution organique est séchée et le solvant est évaporé. On isole une huile incolore (rendement quantitatif).

Huile $[\alpha]_D^{20}$ = - 50,74 (C = 1,58 g/100 ml, méthanol).

c) Chlorhydrate de l'alpha-(tétrahydro-4,5, 6,7 thièno(3,2-c) pyridyl-5) (chloro-2 phényl)-acétate de méthyle lévogyre (SR 25989 A).

Préparé selon le mode opératoire décrit dans l'exemple 1d). Rendement : 94 %.

Cristaux blancs, F = 117 ° C, $[\alpha]_D^{20}$ = - 62,56 (C = 1,80 g/100 ml méthanol).

d) Hydrogénosulfate de l'alpha-(tétrahydro-4,5,6,7 thiéno(3,2-c) pyridyl-5) (chloro-2 phényl)-acétate de méthyle lévogyre (SR 25989 C).

On basifie 70 g (0,126 mole) de camphosulfonate SR 25989 B obtenu selon a) ci-dessus avec une solution aqueuse saturée de bicarbonate de sodium en présence de dichlorométhane. La phase organique est décantée, séchée sur sulfate et évaporée à sec.

Au résidu repris par 300 ml d'acétone, on ajoute goutte à goutte 7,2 ml (0,126 mole) d'acide sulfurique concentré. Après agitation, on filtre les cristaux formés et on lave à l'acétone. On obtient 47,8 g de cristaux blancs.

F = 182 ° C $[\alpha]_D^{20}$ = -51,61 (C=2,044 g/100 ml méthanol).

Analyse (C,H,N) conforme.

<u>ETUDE PHARMACOLOGIQUE</u>

L'activité antiagrégante et la toxicité des nouveaux composés ont été comparées à celles du mélange racémique, décrit dans le brevet français n° 82.12599 (publication n° 2 530 247).

Dans ce qui suit, on décrit les résultats de cette étude qui met en évidence un autre avantage de l'invention, à savoir que les sels de l'isomère dextrogyre ont un meilleur indice thérapeutique que le sel du mélange racémique; en effet, l'isomère lévogyre ne présente pratiquement pas d'activité antiagrégante plaquettaire et sa toxicité est nettement supérieure à celle de son homologue dextrogyre.

Les activités antiagrégante plaquettaire et antithrombotique des composés ont été étudiées chez le rat par des méthodes classiques.

On a déterminé, ex-vivo, l'activité sur l'agrégation des plaquettes induite par l'ADP ou par le collagène.

Les produits en solution dans de l'éthanol (200 mg/ml) dilués dans de l'eau contenant de la gomme arabique (5%-p/v) ont été administrés par voie orale à des lots de 5 rats femelles de souche CD-COBS, pesant 250-300 g, à raison de 10 ml de suspension par kilogramme, deux heures avant le prélèvement sanguin.

Les prélèvements sur citrate de sodium en solution aqueuse à 3,8% (1 vol/9 volumes de sang) ont été effectués, sur les animaux anesthésiés à l'éther diéthylique, par ponction à l'aorte abdominale. Le plasma riche en plaquettes a ensuite été isolé par centrifugation à 200 g pendant 10 minutes.

L'agrégation est induite par l'addition de 2 µl de solution agrégante pour 400 µl de plasma riche en plaquettes. Les solutions agrégantes utilisées ont été : une solution aqueuse d'ADP commercialisé par Boehringer Mannheim de concentration 500 µM (concentration finale 2,5 µM), et une solution de collagène commercialisé par Sigma (type 1) à 0,25 g/100 ml dans l'acide acétique à 3% (v/v) (concentration finale 12,5 µg/ml).

L'agrégation des plaquettes a été suivie, selon la méthode décrite par G.V.R. Born dans Nature <u>194</u> p. 927 (1967), avec un agrégomètre Coultronics [R] à la température de 37 ° C, avec une agitation de 900 tpm.

Pour l'agrégation à l'ADP, l'agrégomètre fournit une courbe représentant l'agrégation plaquettaire mesurée par un changement de densité optique. La hauteur de cette courbe est définie comme hauteur

d'agrégation. Le pourcentage d agrégation est le rapport entre la hauteur d'agrégation mesurée et la hauteur correspondant à 100% d'agrégation x 100. Le pourcentage d'inhibition est déterminé par le rapport:

$$\frac{\text{Hauteur d'agrégation témoin-hauteur agrégation produit}}{\text{Hauteur agrégation témoin}} \times 100$$

Les résultats obtenus sur l'agrégation à l'ADP, pour le chlorhydrate de mélange racémique (PCR 4099), les hydrogénosulfates des isomères dextrogyre (SR 25990 C) et lévogyre (SR 25989 C) d'une part, le PCR 4099 et les chlorhydrates des isomères dextrogyre (SR 25990 A) et lévogyre (SR 25989 A), d'autre part, sont indiqués dans le tableau I; ils démontrent que l'isomère lévogyre est inactif, et que l'isomère dextrogyre est au moins aussi actif que le racémique.

TABLEAU I

| PRODUIT | DOSE mg/Kg P.O | QUANTITE de base administrée | % D'AGREGATION | % INHIBITION | P** |
|---|---|---|---|---|---|
| Témoins | | | 42,4 +/-1,5 | | |
| PCR 4099 (racémique) | 4,48<br>8,97<br>17,9 | 3,84<br>7,69<br>15,38 | 29,8 +/-2,4<br>17,2 +/-2,2<br>11,1 +/-2,3 | 30<br>59<br>74 | 0,01<br>0,001<br>0,001 |
| SR 25989C | 20<br>40 | 15,38<br>30,76 | 41,0 +/-1,5<br>37,1 +/-1,7 | 3<br>13 | n.s<br>n.s |
| SR 25990C | 1,25<br>2,5<br>5<br>10 | 0,96<br>1,92<br>3,84<br>7,69 | 39,4 +/-1,3<br>28,4 +/-2,3<br>14,0 +/-1,6<br>8,5 +/-1,6 | 7<br>33<br>67<br>80 | n.s<br>0,01<br>0,001<br>0,001 |

* moyenne des résultats +/- écart standard moyen (ESM)
** test de Student

Pour l'agrégation au collagène, le pourcentage d'inhibition est la différence des pentes des courbes représentant la variation de densité optique en fonction du temps pour le témoin et le produit à tester divisée par la pente pour le témoin, multiplié par 100. Les résultats figurant dans le tableau II démontrent encore que seul l'isomère dextrogyre est actif, tandis que les sels ont des activités comparables.

TABLEAU II

| PRODUIT | DOSE mg/Kg P.O | QUANTITE de base administrée | PENTE | % INHIBITION | P** |
|---|---|---|---|---|---|
| Témoins | | | 4,8 +/-0,3 | | |
| PCR 4099 (racémique) | 4,48 | 3,84 | 3,6 +/-0,2 | 25 | 0,05 |
| | 8,97 | 7,69 | 2,7 +/-0,3 | 44 | 0,01 |
| | 17,9 | 15,38 | 1,5 +/-0,3 | 69 | 0,001 |
| SR 25989C | 20 | 15,38 | 4,3 +/-0,2 | 10 | n.s |
| | 40 | 30,76 | 4,0 +/-0,2 | 17 | n.s |
| SR 25990C | 1,25 | 0,96 | 4,5 +/-0,3 | 6 | n.s |
| | 2,5 | 1,92 | 4,1 +/-0,2 | 15 | n.s |
| | 5 | 3,84 | 2,3 +/-0,1 | 52 | 0,001 |
| | 10 | 7,69 | 1,7 +/-0,3 | 65 | 0,001 |

** test de Student
n.s non significatif.

On a aussi étudié l'activité antithrombotique des composés dans le test de la thrombose veineuse sur la vrille décrit par Kumada T. et Coll. dans Thromb. Res 18 p. 189 (1980).

Les rats femelles de même type que précédemment, à raison de 10 animaux par lot, ont été anesthésiés à l'éther diéthylique et leur veine cave isolée après incision abdominale.

On a introduit dans la lumière de cette veine, juste au-dessous de la bifurcation rénale en descendant vers les veines iliaques, sans léser la paroi, une vrille métallique constituée d'un bourre-pâte de dentiste de 21 mm de long, commercialisée par Dyna (France) taille n° 30; la vrille est implantée sur une longueur de 19 à 20 mm et dépasse de 1 mm l'extérieur et l'abdomen refermé.

Les thrombi se forment rapidement, et cinq heures plus tard, sous anesthésie au pentobarbital, l'abdomen est réouvert et des ligatures sont posées en amont et aval de la vrille qui est retirée après incision longitudinale de la veine et le thrombus isolé est pesé.

Les résultats qui figurent dans le tableau III montrent que l'isomère lévogyre est inactif dans ce test, contrairement à l'isomère dextrogyre et au racémique.

TABLEAU III

| PRODUIT | DOSE mg/Kg P.O administrée | QUANTITE de base | POIDS des thrombi * | VARIATION % | P** |
|---|---|---|---|---|---|
| Témoins | | | 3,9 +/-0,3 | | |
| PCR 4099 (racémique) | 4,48 | 3,84 | 2,17 +/-0,24 | 44 | 0,001 |
| | 8,97 | 7,69 | 1,39 +/-0,15 | 64 | 0,001 |
| | 17,9 | 15,38 | 1,00 +/-0,19 | 74 | 0,001 |
| SR 25989C | 40 | 30,76 | 4,17 +/-0,42 | -7 | n.s |
| SR 25990C | 1,25 | 0,96 | 3,11 +/-0,32 | 20 | n.s |
| | 2,5 | 1,92 | 2,29 +/-0,22 | 41 | 0,01 |
| | 5 | 3,84 | 1,71 +/-0,24 | 56 | 0,01 |
| | 10 | 7,69 | 1,26 +/-0,19 | 67 | 0,01 |
| | 20 | 15,38 | 1,20 +/-0,13 | 69 | 0,01 |

* = poids des thrombi en mg +/- écart standard moyen
P = test U de Kruskal-Wallis

Pour l'étude toxicologique, les composés ont été administrés par voie orale sous forme de suspension dans un même volume d'eau additionnée de gomme arabique à 10% (p/v) à des lots de 10 rats femelles de souche Sprague Dawley, pesant 120 à 135 grammes, à jeun.

Le nombre de morts a été déterminé 14 jours après l'administration du produit à étudier. Les doses léthales alors déterminées, exprimées en poids du sel administré, figurent dans le tableau IV; ces résultats montrent d'une part que la toxicité du mélange racémique est proche de celle de l'isomère lévogyre, tandis que l'isomère dextrogyre est nettement moins toxique, et que d'autre part, la toxicité depend de la nature de l'acide utilisé pour la salification.

TABLEAU IV

| PRODUITS | D 10 | D 50 ( ) | D 90 | DOSE LETHALE ABSOLUE |
|---|---|---|---|---|
| PCR 4099 (racémique) | 1318 | 1615 (1448-1747) | 1979 | 2000 |
| SR 25989 A | 1259 | 1702 (1443-1797) | 2299 | 2000 |
| SR 25990 A | 3055 | 4316 (3569-5705) | 6137 | 5000 |
| SR 25990 C | 2257 | 2591 (2372-2805) | 2974 | 4000 |
| ( ) = intervalle de confiance | | | | |

**Revendications**

**Revendications pour les Etats contractants suivants : DE, AT, BE, FR, GR, IT, NL, GB, LU, SE, CH, LI**

**1.** Hydrogénosulfate de l'isomère dextrogyre de l'alpha-(tétrahydro-4,5,6,7 thiéno(3,2-c) pyridyl-5) (chloro-2 phényl)-acétate de méthyle.

**2.** Procédé de préparation du composé selon la revendication 1, caractérisé en ce que l'on salifie dans l'acétone l'alpha-(tétrahydro-4,5,6,7 thiéno (3,2-c) pyridyl-5)(chloro-2 phényl)-acétate de méthyle racémique par l'acide camphosulfonique-10 lévogyre, on effectue des recristallisations successives du sel dans l'acétone jusqu'à l'obtention d'un produit de pouvoir rotatoire constant, puis on libère l'isomère dextrogyre de son sel par une base, et on effectue sa salification avec l'acide sulfurique.

**3.** Composition pharmaceutique caractérisée en ce qu'elle contient, à titre de principe actif, un composé selon la revendication 1.

**4.** Composition selon la revenddication 3, caractérisée en ce que chaque dose unitaire contient de 0,001 g à 0,100 g de principe actif.

**Revendications pour l'Etat contractant suivant : ES**

**1.** Procédé de préparation de l'hydrogénosulfate de l'isomère dextrogyre de l'alpha-(tétrahydro-4,5,6,7 thiéno (3,2-c) pyridyl-5) (chloro-2 phényl)-acétate de méthyle, caractérisé en ce que l'on salifie dans l'acétone l'alpha-(tétrahydro-4,5,6,7 thiéno (3,2-c) pyridyl-5) (chloro-2 phényl)-acétate de méthyle racémique par l'acide camphosulfonique-10 lévogyre, on effectue des recristallisations successives du sel dans l'acétone jusqu'à l'obtention d'un produit de pouvoir rotatoire constant, puis on libère l'isomère dextrogyre de son sel par une base, et on effectue sa salification avec l'acide sulfurique.

**2.** Procédé de préparation d'une composition pharmaceutique, caractérisé en ce que l'on met sous forme pharmaceutiquement acceptable un composé obtenu selon la revendication 1.

**Claims**

**Claims for the following Contracting States : DE, AT, BE, FR, GR, IT, NL, GB, LU, SE, CH, LI**

**1.** Hydrogen sulphate of the dextrorotatory isomer of alpha-(4,5,6,7-tetrahydrothieno[3,2-c]pyrid-5-yl)(2-chlorophenyl)methyl acetate.

**2.** Process for preparing the compound according to claim 1, characterised in that racemic alpha-(4,5,6,7-tetrahydrothieno[3,2-c]pyrid-5-yl)(2-chlorophenyl)methyl acetate is converted into a salt in acetone with laevorotatory 10-camphosulpbonic acid, successive recrystallisations of the salt are carried out in acetone until a product having a constant rotatory power is obtained, then the dextrorotatory isomer is

liberated from its salt by a base and converted into a salt with sulphuric acid.

**3.** Pharmaceutical composition, characterised in that it contains, as active principle, a compound according to claim 1.

**4.** Composition according to claim 3, characterised in that each dosage unit contains from 0.001 g to 0.100 g of active principle.

**Claims for the following Contracting State : ES**

**1.** Process for preparing the hydrogen sulphate of the dextrorotatory isomer of alpha-(4,5,6,7-tetrahydrothieno[3,2-c]pyrid-5-yl)(2-chlorophenyl)methyl acetate, characterised in that racemic alpha-(4,5,6,7-tetrahydrothieno[3,2-c]pyrid-5-yl)(2-chlorophenyl)methyl acetate is converted into a salt in acetone with laevorotatory 10-camphosulphonic acid, successive recrystallisations of the salt are carried out in acetone until a product having a constant rotatory power is obtained, then the dextrorotatory isomer is liberated from its salt by a base and converted into a salt with sulphuric acid.

**2.** Process for preparing a pharmaceutical composition, characterised in that a compound obtained according to claim 1 is put into a pharmaceutically acceptable form.

**Patentansprüche**
**Patentansprüche für folgende Vertragsstaaten : DE, AT, BE, FR, GR, IT, NL, GB, LU, SE, CH, LI**

**1.** Hydrogensulfat des rechtsdrehenden Isomeren des $\alpha$-(4,5,6,7-Tetrahydro-thieno[3,2-c]pyrid-5-yl)-(2-chlor-phenyl)-essigsäuremethylesters.

**2.** Verfahren zur Herstellung der Verbindung nach Anspruch 1, **dadurch gekennzeichnet,** daß man den racemischen $\alpha$-(4,5,6,7-Tetrahydro-thieno[3,2-c]pyrid-5-yl)-(2-chlor-phenyl)-essigsäuremethylester mit linksdrehender 10-Camphersulfonsäure in Aceton in das Salz überführt, aufeinanderfolgende Umkristallisationen des Salzes in Aceton bewirkt, bis man ein Produkt mit konstantem Drehwert erhält und dann das rechtsdrehende Isomere mit einer Base aus seinem Salz freisetzt und sein Salz mit Schwefelsäure bildet.

**3.** Pharmazeutische Zubereitung, **dadurch gekennzeichnet,** daß sie als Wirkstoff eine Verbindung nach Anspruch 1 enthält.

**4.** Zubereitung nach Anspruch 3, **dadurch gekennzeichnet,** daß jede Einheitsdosis 0,001 bis 0,100 g des Wirkstoffs enthält.

**Patentansprüche für folgenden Vertragsstaat : ES**

**1.** Verfahren zur Herstellung des Hydrogensulfats des rechtsdrehenden Isomeren des $\alpha$-(4,5,6,7-Tetrahydro-thieno[3,2-c]pyrid-5-yl)-(2-chlorphenyl)-essigsäuremethylesters, **dadurch gekennzeichnet,** daß man den racemischen $\alpha$-(4,5,6,7-Tetrahydro-thieno[3,2-c]pyrid-5-yl)-(2-chlorphenyl)-essigsäuremethylester mit linksdrehender 10-Camphersulfonsäure in Aceton in das Salz überführt, aufeinander folgende Umkristallisationen des Salzes in Aceton bewirkt, bis man ein Produkt mit konstantem Drehwert erhält und dann das rechtsdrehende Isomere mit einer Base aus seinem Salz freisetzt und sein Salz mit Schwefelsäure bildet.

**2.** Verfahren zur Herstellung einer pharmazeutischen Zubereitung, **dadurch gekennzeichnet,** daß man eine nach Anspruch 1 erhaltene Verbindung in eine pharmazeutisch annehmbare Form bringt.